# EUROPEAN PATENT SPECIFICATION

(11) **EP 1 708 997 B1**
(45) Date of publication and mention of the grant of the patent: **21.10.2009**
(21) Application number: 04798681.5
(22) Date of filing: 25.11.2004
(51) Int. Cl.: C07D 209/30

(54) **1-ALKYL-3-THIO-SUBSTITUTED INDOLE-2-ALKYNOIC ACIDS USEFUL FOR THE TREATMENT FOR ALZHEIMER'S DISEASE AND RELATED CONDITIONS**
FÜR DIE BEHANDLUNG VON ALZHEIMER-KRANKHEIT UND ÄHNLICHEN LEIDEN GEEIGNETE 1-ALKYL-3-THIOSUBSTITUIERTE INDOL-2-ALKINSÄUREN
ACIDES INDOLE-2-ALKYNOIQUES 1-ALKYL-3-THIO-SUBSTITUES UTILISES DANS LE TRAITEMENT DE LA MALADIE D'ALZHEIMER ET LES ETATS ASSOCIES

(30) Priority: 03.12.2003 US 526649
(43) Date of publication of application: 11.10.2006
(73) Proprietor: Merck and Co., Inc., Rahway, NJ 07065 (US); MERCK SHARP & DOHME LTD., Hoddesdon, Hertfordshire EN11 9NU (GB)
(72) Inventor: BEHER, Dirk, Harlow, Essex, CM20 2QR (GB); BETTATI, Michela, Harlow, Essex, CM20 2QR (GB); CHURCHER, Ian, Harlow, Essex, CM20 2QR (GB); MUNZO, Benito, Rahway, NJ 07065-0907 (US); PRASIT, Petpiboon, Rahway, NJ 07065-0907 (US); QUDDUS, Abdul, Harlow, Essex, CM20 2QR (GB); STOCK, Nicholas, S., Rahway, NJ 07065-0907 (US); WRIGLEY, Jonathan, D.J., Harlow, Essex, CM20 2QR (GB)
(74) Representative: Buchan, Gavin MacNicol
(86) International application number: PCT/GB2004/004985
(87) International publication number: WO 2005/054193

(56) References cited:
- WO-A1-00/02561
- US-A- 5 081 138
- US-A1- 2003 082 108
- FRENETTE R ET AL: "Substituted indoles as potent and orally active 5-lipoxygenase activating protein (FLAP) inhibitors" BIOORGANIC & MEDICINAL CHEMISTRY LETTERS, OXFORD, GB, vol. 9, no. 16, 16 August 1999 (1999-08-16), pages 2391-2396, XP004174197 ISSN: 0960-894X

## Description

This invention relates to the use of methods and materials for use in therapeutic treatment of the human body. In particular, it provides methods of treating diseases associated with the deposition of β-amyloid peptide in the brain, such as Alzheimer's disease, or of preventing or delaying the onset of dementia associated with such diseases.

Alzheimer's disease (AD) is the most prevalent form of dementia. Its diagnosis is described in the Diagnostic and Statistical Manual of Mental Disorders, 4^{th} ed., published by the American Psychiatric Association (DSM-IV). It is a neurodegenerative disorder, clinically characterized by progressive loss of memory and general cognitive function, and pathologically characterized by the deposition of extracellular proteinaceous plaques in the cortical and associative brain regions of sufferers. These plaques mainly comprise fibrillar aggregates of β-amyloid peptide (Aβ). Aβ is formed from amyloid precursor protein (APP) via separate intracellular proteolytic events involving the enzymes β-secretase and γ-secretase. Variability in the site of the proteolysis mediated by γ-secretase results in Aβ of varying chain length, e.g. Aβ(1-38), Aβ(1-40) and Aβ(1-42). N-terminal truncations such as Aβ(4-42) are also found in the brain, possibly as a result of variability in the site of proteolysis mediated by β-secretase. For the sake of convenience, expressions such as "Aβ(1-40)" and "Aβ(1-42)" as used herein are inclusive of such N-terminal truncated variants. After secretion into the extracellular medium, Aβ forms initially-soluble aggregates which are widely believed to be the key neurotoxic agents in AD (see Gong et al, PNAS,100 (2003), 10417-22), and which ultimately result in the insoluble deposits and dense neuritic plaques which are the pathological characteristics of AD.

Other dementing conditions associated with deposition of Aβ in the brain include cerebral amyloid angiopathy, hereditary cerebral haemorrhage with amyloidosis, Dutch-type (HCHWA-D), multi-infarct dementia, dementia pugilistica and Down syndrome.

Various interventions in the plaque-forming process have been proposed as therapeutic treatments for AD (see, for example, Hardy and Selkoe, Science, 297 (2002), 353-6). One such method of treatment that has been proposed is that of blocking or attenuating the production of Aβ, for example by inhibition of β- or γ-secretase. It has also been reported that inhibition of glycogen synthase kinase-3 (GSK-3), in particular inhibition of GSK-3α, can block the production of Aβ (see Phiel et al, Nature, 423 (2003), 435-9).

Other proposed methods of treatment include administering a compound which blocks the aggregation of Aβ, and administering an antibody which selectively binds to Aβ.

Another proposed treatment is that of modulation of the action of γ-secretase so as to selectively attenuate the production of Aβ(1-42). This results in preferential secretion of the shorter chain isoforms of Aβ, which are believed to have a reduced propensity for self-aggregation and plaque formation, and hence are more easily cleared from the brain, and/or are less neurotoxic. Compounds showing this effect include certain non-steroidal antiinflammatory drugs (NSAIDs) and their analogues (see WO 01/78721 and US 2002/0128319 and Weggen et al Nature, 414 (2001) 212-16; Morihara et al, J. Neurochem., 83 (2002), 1009-12; and Takahashi et al, J. Biol. Chem., 278 (2003), 18644-70). Certain compounds which modulate the activity of PPARα and/or PEARδ are also reported to have the effect of lowering Aβ(1-42) (WO 02/100836). NSAID derivatives capable of releasing nitric oxide have been reported to show improved anti-neuroinflammatory effects and/or to reduce intracerebral Aβ deposition in animal models (WO 02/092072; Jantzen et al, J. Neuroscience, 22 (2002), 226-54). US 2002/0015941 teaches that agents which potentiate capacitative calcium entry activity can lower Aβ(1-42).

US 5,081,138 discloses a broad class of 1-benzyl-3-thio-substituted indole-2-alkanoic acids as inhibitors of leukotriene biosynthesis, useful in treatment of asthma, allergies, inflammation, diarrhoea, hypertension, angina, platelet aggregation, cerebral spasm, premature labour, spontaneous abortion, dysmenorrhea and migraine. There is no disclosure or suggestion of any effect on the secretion of Aβ, or of any utility in the treatment or prevention of AD or any other disorders associated with deposition of Aβ in the brain.

WO 00/02561 discloses the use of modulators of the phospholipase A2 pathway (including MK-886) for regulation of the Aβ pro-inflammatory pathway.

It has now been found that certain 1-alkyl-3-thio-substituted indole-2-alkanoic acids have the desirable property of selectively inhibiting production of Aβ(1-42).

According to the present invention there is provided the use, for the manufacture of a medicament for selective attenuation of the production of the (1-42) isoform of Aβ, of a compound of Formula I 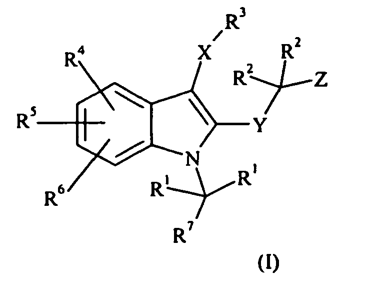 wherein each R¹ is independently H, C₁₋₆alkyl or C₃₋₆cycloalkyl;
each R² is independently H or C₁₋₆alkyl, or the two R² groups together complete a C₃₋₆cycloalkyl group;
R³ represents a hydrocarbon group of up to 10 carbon atoms optionally bearing up to 2 substituents selected from halogen, CN, CF₃, OCF₃, C₁₋₄alkoxy, C₁₋₄alkylthio, CO₂H, C₁₋₄alkoxycarbonyl, C₁₋₄alkylcarbonyl and C₁₋₄alkylcarbonyloxy; or R³ represents a heteroaryl group of 5 or 6 ring atoms which is optionally benzo-fused, optionally bearing up to 2 substituents selected from C₁₋₄alkyl, halogen, CN, CF₃, OCF₃, C₁₋₄alkoxy, C₁₋₄alkylthio, CO₂H, C₁₋₄alkoxycarbonyl, C₁₋₄alkylcarbonyl and C₁₋₄ alkylcarbonyloxy;
R⁴, R⁵ and R⁶ independently represent H, halogen, CN, CF₃, OCF₃, C₁₋₄alkoxy, C₁₋₄alkylthio, CO₂H, C₁₋₄alkoxycarbonyl, C₁₋₄alkylcarbonyl, C₁₋₄alkylcarbonyloxy or hydrocarbon of up to 7 carbon atoms optionally bearing a substituent selected from halogen, CN, CF₃, OCF₃, C₁₋₄alkoxy, C₁₋₄alkylthio, CO₂H, C₁₋₄alkoxycarbonyl, C₁₋₄alkylcarbonyl and C₁₋₄alkylcarbonyloxy;
R⁷ represents a hydrocarbon group of up to 10 carbon atoms optionally bearing up to 2 substituents selected from halogen, CN, CF₃, OCF₃, C₁₋₄alkoxy, C₁₋₄alkylthio, CO₂H, C₁₋₄alkoxycarbonyl, C₁₋₄alkylcarbonyl and C₁₋₄alkylcarbonyloxy;
X represents O or S;
Y represents a bond, CH₂, CH₂CH₂, CH₂CH₂CH₂ or CH₂CH₂O; and
Z represents CO₂H or tetrazol-5-yl;
or a pharmaceutically acceptable salt thereof.

The medicament is suitable for administration to patients suffering from disease associated with deposition of Aβ in the brain, for example Alzheimer's disease (AD), cerebral amyloid angiopathy, HCHWA-D, multi-infarct dementia, dementia pugilistica or Down syndrome, in particular AD.

The invention also finds use in treating, preventing or delaying the onset of dementia associated with Alzheimer's disease, cerebral amyloid angiopathy, HCHWA-D, multi-infarct dementia, dementia pugilistica or Down syndrome.

The compounds of Formula I modulate the action ofy-secretase so as to selectively attenuate production of the (1-42) isoform of Aβ without significantly lowering production of the shorter chain isoforms such as Aβ(1-40). This results in secretion of Aβ which has less tendency to self-aggregate and form insoluble deposits, is more easily cleared from the brain, and/or is less neurotoxic. Therefore, a further use of the invention is for retarding, arresting or preventing the accumulation of Aβ in the brain.

Because the compounds of formula I modulate the activity of γ-secretase, as opposed to suppressing said activity, it is believed that the therapeutic benefits described above will be obtained with a reduced risk of side effects, e.g. those that might arise from a disruption of other signalling pathways (e.g. Notch) which are controlled by γ-secretase.

In one embodiment of the invention, there is provided the use of the compound of formula I for the manufacture of a medicament that is administered to a patient suffering from AD, cerebral amyloid angiopathy, HCHWA-D, multi-infarct dementia, dementia pugilistica or Down syndrome, in particular a patient suffering from AD.

In an alternative embodiment of the invention, there is provided the use of the compound of formula I for the manufacture of a medicament that is administered to a patient suffering from mild cognitive impairment or age-related cognitive decline. A favourable outcome of such treatment is prevention or delay of the onset of AD. Age-related cognitive decline and mild cognitive impairment (MCI) are conditions in which a memory deficit is present, but other diagnostic criteria for dementia are absent (Santacruz and Swagerty, American Family Physician, 63 (2001), 703-13). (See also "The ICD-10 Classification of Mental and Behavioural Disorders", Geneva: World Health Organisation, 1992, 64-5). As used herein, "age-related cognitive decline" implies a decline of at least six months' duration in at least one of: memory and learning; attention and concentration; thinking; language; and visuospatial functioning and a score of more than one standard deviation below the norm on standardized neuropsychologic testing such as the MMSE. In particular, there may be a progressive decline in memory. In the more severe condition MCI, the degree of memory impairment is outside the range considered normal for the age of the patient but AD is not present. The differential diagnosis of MCI and mild AD is described by Petersen et al., Arch. Neurol., 56 (1999), 303-8. Further information on the differential diagnosis of MCI is provided by Knopman et al, Mayo Clinic Proceedings, 78 (2003), 1290-1308. In a study of elderly subjects, Tuokko et al (Arch, Neurol., 60 (2003) 577-82) found that those exhibiting MCI at the outset had a three-fold increased risk of developing dementia within 5 years.

Grundman et al (J. Mol. Neurosci., 19 (2002), 23-28) report that lower baseline hippocampal volume in MCI patients is a prognostic indicator for subsequent AD. Similarly, Andreasen et al (Acta Neurol. Scand, 107 (2003) 47-51) report that high CSF levels of total tau, high CSF levels of phospho-tau and lowered CSF levels of Afi42 are all associated with increased risk of progression from MCI to AD.

Within this embodiment, the compound of Formula I is advantageously administered to patients who suffer impaired memory function but do not exhibit symptoms of dementia. Such impairment of memory function typically is not attributable to systemic or cerebral disease, such as stroke or metabolic disorders caused by pituitary dysfunction. Such patients may be in particular people aged 55 or over, especially people aged 60 or over, and preferably people aged 65 or over. Such patients may have normal patterns and levels of growth hormone secretion for their age. However, such patients may possess one or more additional risk factors for developing Alzheimer's disease. Such factors include a family history of the disease; a genetic predisposition to the disease; elevated serum cholesterol; and adult-onset diabetes mellitus.

In a particular embodiment of the invention, the compound of Formula I is administered to a patient suffering from age-related cognitive decline or MCI who additionally possesses one or more risk factors for developing AD selected from: a family history of the disease; a genetic predisposition to the disease; elevated serum cholesterol; adult-onset diabetes mellitus; elevated baseline hippocampal volume; elevated CSF levels of total tau; elevated CSF levels of phospho-tau; and lowered CSF levels of Ap(l-42).

A genetic predisposition (especially towards early onset AD) can arise from point mutations in one or more of a number of genes, including the APP, presenilin-1 and presenilin-2 genes. Also, subjects who are homozygous for the ε4 isoform of the apolipoprotein E gene are at greater risk of developing AD.

The patient's degree of cognitive decline or impairment is advantageously assessed at regular intervals before, during and/or after a course of treatment in accordance with the invention, so that changes therein may be detected, e.g. the slowing or halting of cognitive decline. A variety of neuropsychological tests are known in the art for this purpose, such as the Mini-Mental State Examination (MUSE) with norms adjusted for age and education (Folstein et al., J. Psych. Res.,12 (1975), 196-198, Anthony et al., Psychological Med.,12 (1982), 397-408; Cockrell et al., Psychopharmacology, 24 (1988), 689-692; Crum et al., J. Am. Med. Assoc'n. 18 (1993), 2386-2391). The MMSE is a brief, quantitative measure of cognitive status in adults. It can be used to screen for cognitive decline or impairment, to estimate the severity of cognitive decline or impairment at a given point in time, to follow the course of cognitive changes in an individual over time, and to document an individual's response to treatment. Another suitable test is the Alzheimer Disease Assessment Scale (ADAS), in particular the cognitive element thereof (ADAS-cog) (See Rosen et al., Am. J. Psychiatry, 141 (1984), 1356-64).

Where a variable occurs more than once in formula I or in a substituent thereof, the individual occurrences of that variable are independent of each other, unless otherwise specified.

As used herein, the expression "hydrocarbon group" refers to groups consisting solely of carbon and hydrogen atoms. Such groups may comprise linear, branched or cyclic structures, singly or in any combination consistent with the indicated maximum number of carbon atoms, and may be saturated or unsaturated, including aromatic when the indicated maximum number of carbon atoms so permits unless otherwise indicated.

As used herein, the expression "C₁₋ₓalkyl" where x is an integer greater than 1 refers to straight-chained and branched alkyl groups wherein the number of constituent carbon atoms is in the range 1 to x. Particular alkyl groups are methyl, ethyl, n-propyl, isopropyl and t-butyl. Derived expressions such as "C₂₋₆alkenyl", "hydroxyC₁₋₆ alkyl", "heteroarylC₁₋₆alkyl", "C₂₋₆alkynyl" and "C₁₋₆alkoxy" are to be construed in an analogous manner. Most suitably, the number of carbon atoms in such groups is not more than 6.

The term "halogen" as used herein includes fluorine, chlorine, bromine and iodine, of which fluorine and chlorine are preferred.

The term "heteroaryl" as used herein refers to an aromatic ring in which one or more of the ring atoms is other than carbon. Preferably, said one or more ring atoms are selected from N, O and S.

For use in medicine, the compounds of formula I may be in the form of pharmaceutically acceptable salts. Other salts may, however, be useful in the preparation of the compounds of formula I or of their pharmaceutically acceptable salts. Suitable pharmaceutically acceptable salts of the compounds of this invention include acid addition salts which may, for example, be formed by mixing a solution of the compound according to the invention with a solution of a pharmaceutically acceptable acid such as hydrochloric acid, sulphuric acid, methanesulphonic acid, benzenesulphonic acid, fumaric acid, maleic acid, succinic acid, acetic acid, benzoic acid, oxalic acid, citric acid, tartaric acid, carbonic acid or phosphoric acid. Alternatively, where the compound of the invention carries an acidic moiety, a pharmaceutically acceptable salt may be formed by neutralisation of said acidic moiety with a suitable base. Examples of pharmaceutically acceptable salts thus formed include alkali metal salts such as sodium or potassium salts; ammonium salts; alkaline earth metal salts such as calcium or magnesium salts; and salts formed with suitable organic bases, such as amine salts (including pyridinium salts) and quaternary ammonium salts.

Where the compounds according to the invention have at least one asymmetric centre, they may accordingly exist as enantiomers. Where the compounds according to the invention possess two or more asymmetric centres, they may additionally exist as diastereoisomers. It is to be understood that all such isomers and mixtures thereof in any proportion are encompassed within the scope of the present invention.

In formula I, X represents O or S. In a preferred embodiment, X represents S.

Y represents a bond, CH₂, CH₂CH₂, CH₂CH₂CH₂ or CH₂CH₂O. Typically, Y represents a bond, CH₂ or CH₂CH₂O, and preferably represents CH₂.

Z represents CO₂H or tetazol-5-yl. Preferably, Z represents CO₂H.

In Formula I, each R¹ is independently H, C₁₋₆ alkyl or C₃₋₆ cycloalkyl. Examples include H, methyl, ethyl, propyl and cyclopropyl. Preferably at least one R¹ is H. In one preferred embodiment, both R¹ groups are H. In another preferred embodiment, one R¹ is H and the other is methyl, ethyl or propyl, preferably methyl.

Each R² is independently H or C₁₋₆alkyl (such as methyl or ethyl), or the two R² groups together complete a cycloalkane of 3-6 members (such as cyclopentane or cyclohexane). Preferably, at least one R² is other than H, and most preferably neither R² is H. In a preferred embodiment both R² groups are methyl.

R³ represents hydrocarbon of up to 10 carbon atoms, optionally substituted as defined previously, or heteroaryl of 5 to 6 atoms, optionally benzo-fused, optionally substituted as defined previously. Typical hydrocarbon groups include alkyl (especially branched alkyl, such as t-butyl), cycloalkyl (such as cyclohexyl), phenyl, phenyl-substituted alkyl (such as benzyl), and alkyl-substituted phenyl (such as toluyl or xylyl). Preferred substituents (where present) include halogen, especially Cl. Typical heteroaryl rings include pyridine, quinoline, isoquinoline, thiazole and benzothiazole, on which the preferred substituents (where present) include halogen, especially Cl.

In a preferred embodiment, R³ is selected from alkyl, cycloalkyl, alkyl-substituted phenyl and chloro-substituted phenyl.

Specific examples of groups represented by R³ include t-butyl, cyclohexyl, phenyl, 2,5-dimethylphenyl, 2,6-dimethylphenyl, 3,5-dimethylphenyl, 3,4-dimethylphenyl, 2,4-dimethylphenyl, 4-chlorophenyl, 3,4-dichlorophenyl and 5-chlorobenzothiazol-2-yl.

R⁴, R⁵ and R⁶ are perfectly selected from H, halogen, CN, CF₃, OCF₃, C₁₋₄ alkoxy, C₁₋₆alkyl and optionally-substituted phenyl. Preferably, at least one of R⁴, R⁵ and R⁶ is other than H. When two or more of R⁴, R⁵ and R⁶ are other than H, they are very aptly selected from C₁₋₆alkyl and halogen. R⁴, R⁵ and R⁶ may occupy any of the available ring positions, but in one preferred embodiment, two of R⁴, R⁵ and R⁶ are H and the third is attached to the 5-position. Specific examples of groups represented by R⁴, R⁵ and R⁶ include H, methyl, isopropyl, Cl, Br, F, CF₃, OCF₃ and phenyl.

Examples of hydrocarbon groups represented by R⁷ include alkyl (e.g. of up to 6 carbon atoms, such as methyl, ethyl, propyl or butyl), which is optionally substituted as defined previously; alkenyl (e.g. of up to 6 carbon atoms, such as vinyl or allyl), cycloalkyl (e.g. of up to 6 carbon atoms, such as cyclohexyl), and optionally substituted phenyl. Phenyl groups represented by R⁷ preferably bear at least one substituent, and preferably not more than two substituents. If more than two substituents are present, at least one of them must be C₁₋₄alkyl. When a single substituent is present, it is preferably (but not necessarily) in the 4-position. Examples of preferred substituents include C₁₋₄alkyl (such as methyl, ethyl, propyl and butyl, especially t-butyl); halogen (especially Cl and F); CN; CF₃; OCF₃; CO₂H and C₁₋₄alkylthio (especially methylthio). Specific examples of groups represented by R⁷ include methyl, methoxymethyl, vinyl, cyclohexyl, phenyl, 4-chlorophenyl,4-trifluoromethylphenyl, 4-fluorophenyl, 2,4-dichlorophenyl, 2,4-difluorophenyl, 4-trifluoromethoxyphenyl, 4-t-butylphenyl, 3,5-bis(trifluoromethyl)phenyl, 4-methylthiophenyl, 4-cyanophenyl, 3-trifluoromethylphenyl and 3-carboxyphenyl.

Specific examples suitable for use in the invention include the compounds of formula I in which X is S, Y is CH₂, Z is CO₂H, one R¹ group is H, both R² groups are Me (unless otherwise indicated), and the remaining variables are as shown in the following table:

**Table 1**

| **R¹** | **R³** | **R⁴, R⁵, R⁶** | **R⁷** |
|---|---|---|---|
| Me | 2,5-di-Me-Ph | 5-isopropyl | 4-C1-Ph |
| H | 2,5-di-Me-Ph | 5-isopropyl | 4-CF₃-Ph |
| Me | 2,5-di-Me-Ph | 5-isopropyl | Me |
| H | 2,6-di-Me-Ph | 5-isopropyl | 4-CF₃-Ph |
| H | 3,5-di-Me-Ph | 5-isopropyl | 4-CF₃-Ph |
| Me | 2,5-di-Me-Ph | 5-isopropyl | 4-CF₃-Ph |
| H | 2,5-di-Me-Ph | 5-F | 4-CF₃-Ph |
| H | 3,4-di-Me-Ph | 5-isopropyl | 2,4-di-F-Ph |
| H | 2,5-di-Me-Ph | 5-Br | 4-CF₃-Ph |
| H | 2,5-di-Me-Ph | 5-Br | 4-Cl-Ph |
| H | 2,5-di-Me-Ph | 5-C1 | 4-CF₃-Ph |
| H | 2,5-di-Me-Ph | 5-isopropyl | 2,4-di-C1-Ph |
| H | 2,5-di-Me-Ph | 5-OCF₃ | 4-CF₃-Ph |
| H | 2,5-di-Me-Ph | 5-isopropyl | 2,4-di-CF₃-Ph |
| H | 3,5-di-Me-Ph | 5-isopropyl | 4-Cl-Ph |
| Et | 2,5-di-Me-Ph | 5-isopropyl | 4-CF₃-Ph |
| H | 2,5-di-Me-Ph | 5-C1 | 4-Cl-Ph |
| H | 2,5-di-Me-Ph | 5-isopropyl | cyclohexyl |
| H | 2,5-di-Me-Ph | 5-isopropyl | 4-CF₃O-Ph |
| H | 3,4-di-Me-Ph | 5-isopropyl | 4-CF₃-Ph |
| H | 2,5-di-Me-Ph | 5-CF₃ | 4-CF₃-Ph |
| H | 2,5-di-Me-Ph | 5-isopropyl | 2,4-di-F-Ph |
| H | 2,5-di-Me-Ph | 5-CF₃O | 2,4-di-F-Ph |
| H | 2,4-di-Me-Ph | 5-isopropyl | 4-CF₃-Ph |
| H | 2,6-di-Me-Ph | 5-isopropyl | 4-Cl-Ph |
| H | 2,5-di-Me-Ph | 5-isopropyl | Ph |
| H | 2,5-di-Me-Ph | 5-t-butyl | 4-CF₃-Ph |
| H | 2,5-di-Me-Ph | 5-F | 4-Cl-Ph |
| cyclopropyl | 2,5-di-Me-Ph | 5-isopropyl | 4-CF₃-Ph |
| H | 2,5-di-Me-Ph | 5-isopropyl | 4-t-butyl-Ph |
| H | 2,5-di-Me-Ph | 5-isopropyl | 3,5-di-CF₃-Ph |
| H | 2,5-di-Me-Ph | 5-isopropyl | 4-Cl-Ph |
| H | 2,5-di-Me-Ph | 6,7-di-Me | 4-CF₃-Ph |
| H | 2,4-di-Me-Ph | 5-isopropyl | 4-Cl-Ph |
| H | cyclohexyl | 5-isopropyl | 4-Cl-Ph |
| H | 2,5-di-Me-Ph | 5-isopropyl | 4-MeS-Ph |
| H | 2,5-di-Me-Ph | 6,7-di-Me | 4-Cl-Ph |
| H | 2,5-di-Cl-Ph | 5-isopropyl | 2,4-di-F-Ph |
| H | 2,5-di-Cl-Ph | 5-isopropyl | 4-CF₃-Ph |
| H | 2,5-di-Me-Ph | 5,7-di-Me | 4-Cl-Ph |
| H | 2,5-di-Me-Ph | 5-CF₃O | 4-F-Ph |
| H | 2,5-di-Me-Ph | 5-isopropyl | vinyl |
| H | 2,5-di-Me-Ph | 5-Cl | 4-CN-Ph |
| H | 2,5-di-Me-Ph | 5-CF₃O | 3-Cl-Ph |
| H | 2,5-di-Me-Ph | 5-isopropyl | MeOCH₂ |
| H | 2,5-di-Me-Ph | 5-CF₃O | 4-Cl-Ph |
| H | 2,5-di-Me-Ph | 5-isopropyl | 3-CF₃-Ph |
| H | 2,5-di-Me-Ph | 5-F | 4-CN-Ph |
| H | 3,4-di-Cl-Ph | 5-isopropyl | 4-CF₃-Ph |
| H* | t-butyl | 5-isopropyl | 4-Cl-Ph |
| H | 4-Cl-Ph | 5-isopropyl | 4-Cl-Ph |
| H | 3,4-di-Me-Ph | 5-isopropyl | 4-Cl-Ph |
| H | 3,4-di-Cl-Ph | 5-isopropyl | 4-Cl-Ph |
| H | t-butyl | 5-Ph | 4-Cl-Ph |
| H | 2,5-di-Me-Ph | 5-isopropyl | 3-HOOC-Ph |
| H | t-butyl | 5-isopropyl | 4-Cl-Ph |
| H | 5-Cl-benzothiazol-2-yl | 5-isopropyl | 4-Cl-Ph |

| | | | |
|---|---|---|---|
| * the two R² groups together complete a cyclopentyl ring. | | | |

Further specific compounds suitable for use in the invention include compounds of formula I in which each R¹ is H, R⁴ is 5-isopropyl, R⁵ and R⁶ are H, R⁷ is 4-chlorophenyl and the remaining variables are as shown in table 2:

| **R²/R²** | **R³** | **X** | **Y** | **Z** |
|---|---|---|---|---|
| H, H | t-butyl | S | bond | tetrazol-5-yl |
| H, H | t-butyl | S | CH₂CH₂O | CO₂H |
| H, methyl | t-butyl | S | CH₂CH₂O | CO₂H |
| methyl, methyl | sec-butyl | O | CH₂ | CO₂H |

A first subset of the compounds of Formula I is defined by Formula II: 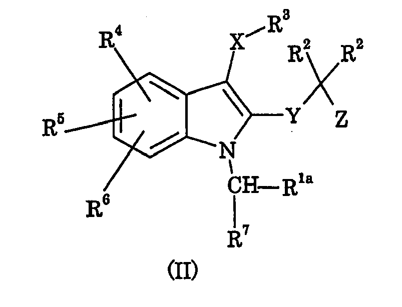 wherein R^{1a} represents C₁₋₆alkyl or C₃₋₆cycloalkyl, and R²-R⁷, X, Y and Z have the same definitions and preferred identities as before.

Compounds of Formula II and the pharmaceutically acceptable salts thereof are believed to be novel and hence constitute a further aspect of the invention. Preferably, X is S, Y is CH₂ and Z is CO₂H.

A second subset of the compounds of Formula I is defined by Formula III: 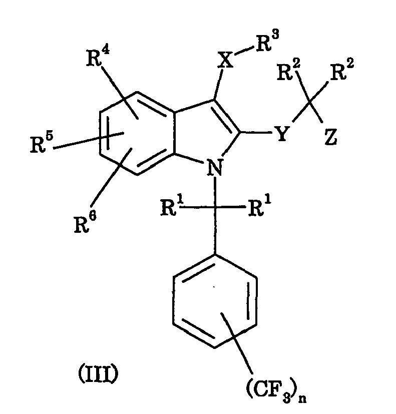 wherein n is 1 or 2 and R¹-R⁶, X, Y and Z have the same definitions and preferred identities as before.

Compounds of formula III and the pharmaceutically acceptable salts thereof are believed to be novel, and hence constitute a further aspect of the invention. In a preferred embodiment, n is 1 and the CF₃ group is in the 4-position. Typically, X is S, Y is CH₂ and Z is CO₂H.

The invention also provides a pharmaceutical composition comprising, in a pharmaceutically acceptable carrier, a compound in accordance with Formula II or Formula III or a pharmaceutically acceptable salt thereof.

The synthesis of compounds of Formula I in which R⁷ is optionally substituted phenyl is described in US 5,081,138. Analogous methods may be used to prepare the corresponding compounds in which R⁷ has its alternative identities.

Since the compounds of Formulae I, II and III may have one or more asymmetric centres, they accordingly may exist in enantiomeric forms. If desired, the individual enantiomers may be isolated in pure form by conventional means. For example, a racemic mixture may be resolved into its component enantiomers by preparative chiral HPLC, or by treatment with an optically pure amine to form diastereomeric salt pairs, separable by fractional crystallisation, from which the optically pure acids may be regenerated. Similarly, a racemic acid may be reacted with an optically pure alcohol or amine to form pairs of diastereomeric esters or amides which may be separated by chromatography or fractional crystallisation and hydrolysed to yield enantiomerically-pure acids. These resolution techniques may equally well be practised on the synthetic precursors of the compounds of Formulae I, II and III and the resulting optically-pure intermediates used to prepare compounds of Formulae I, II and III in optically-pure form.

The compounds of Formulae I, II and III are typically used in the form of pharmaceutical compositions comprising one or more compounds of Formula I, II or III and a pharmaceutically acceptable carrier. Preferably these compositions are in unit dosage forms such as tablets, pills, capsules, powders, granules, sterile parenteral solutions or suspensions, metered aerosol or liquid sprays, drops, ampoules, transdermal patches, auto-injector devices or suppositories; for oral, parenteral, intranasal, sublingual or rectal administration, or for administration by inhalation or insufflation. The principal active ingredient typically is mixed with a pharmaceutical carrier, e.g. conventional tableting ingredients such as corn starch, lactose, sucrose, sorbitol, talc, stearic acid, magnesium stearate and dicalcium phosphate, or gums, dispersing agents, suspending agents or surfactants such as sorbitan monooleate and polyethylene glycol, and other pharmaceutical diluents, e.g. water, to form a homogeneous preformulation composition containing a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This preformulation composition is then subdivided into unit dosage forms of the type described above containing from 0.1 to about 500 mg of the active ingredient of the present invention. Typical unit dosage forms contain from 1 to 100 mg, for example 1, 2, 5, 10, 25, 50 or 100 mg, of the active ingredient. Tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the compositions useful in the present invention may be incorporated for administration orally or by injection include aqueous solutions, liquid- or gel-filled capsules, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyethylene glycol), poly(vinylpyrrolidone) or gelatin.

For treating or preventing Alzheimer's disease, a suitable dosage level is about 0.01 to 250 mg/kg per day, preferably about 0.01 to 100 mg/kg per day, and more preferably about 0.05 to 50 mg/kg of body weight per day, of the active compound. The compounds may be administered on a regimen of 1 to 4 times per day. In some cases, however, a dosage outside these limits may be used.

The compounds of Formula I optionally may be administered in combination with one or more additional compounds known to be useful in the treatment or prevention of AD or the symptoms thereof. Such additional compounds thus include cognition-enhancing drugs such as acetylcholinesterase inhibitors (e.g. donepezil and galanthamine), NMDA antagonists (e.g. memantine) or PDE4 inhibitors (e.g. Ariflo™ and the classes of compounds disclosed in WO 03/018579, WO 01/46151, WO 02/074726 and WO 02/098878). Such additional compounds also include cholesterol-lowering drugs such as the statins, e.g. simvastatin. Such additional compounds similarly include compounds known to modify the production or processing of Aβ in the brain ("amyloid modifiers"), such as compounds which inhibit the secretion of Aβ (including γ-secretase inhibitors, β-secretase inhibitors, and GSK-3α inhibitors), compounds which inhibit the aggregation of Aβ, and antibodies which selectively bind to Aβ.

In this embodiment of the invention, the amyloid modifier may be a compound which inhibits the secretion of Aβ, for example an inhibitor of of γ-secretase (such as those disclosed in WO 01/53255, WO 01/66564, WO 01/70677, WO 01/90084, WO 01/77144, WO 02/30912, WO 02/36555, WO 02/081435, WO 02/081433, WO 03/018543, WO 03/013506, WO 03/013527 and WO 03/014075), or a β-secretase inhibitor (such as those disclosed in WO 03/037325, WO 03/030886, WO 03/006013, WO 03/006021, WO 03/006423, WO 03/006453, WO 02/002122, WO 01/70672, WO 02/02505, WO 02/02506, WO 02/02512, WO 02/02520, WO 02/098849 and WO 02/100820), or any other compound which inhibits the formation or release of Aβ, including those disclosed in WO 98/28268, WO 02/47671, WO 99/67221, WO 01/34639, WO 01/34571, WO 00/07995, WO 00/38618, WO 01/92235, WO 01/77086, WO 01/74784, WO 01/74796, WO 01/74783, WO 01/60826, WO 01/19797, WO 01/27108, WO 01/27091, WO 00/50391, WO 02/057252, US 2002/0025955 and US2002/0022621, and also including GSK-3 inhibitors, particularly GSK-3α inhibitors, such as lithium, as disclosed in Phiel et al, Nature, 423 (2003), 435-9.

Within this embodiment, the amyloid modifier is advantageously a γ-secretase inhibitor, preferred examples of which include a compound of formula XI: 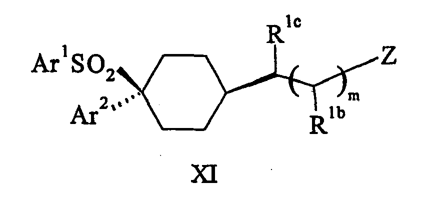 wherein m, Z, R^{1b}, R^{1c}, Ar¹ and Ar² are as defined in WO 03/018543;
or a pharmaceutically acceptable salt thereof.

Such compounds may be prepared as described in WO 03/018543. Preferred examples include those defined by formula XIa: 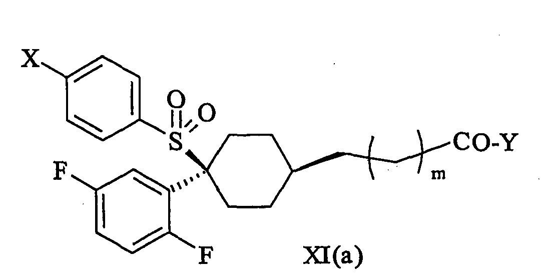 and the pharmaceutically acceptable salts thereof, wherein m is 0 or 1, X is Cl or CF₃, and Y is OH, OC₁₋₆alkyl, NH₂ or NHC₁₋₆alkyl. Particular examples include those in which m is 1 and Y is OH (or the sodium salts thereof), and those in which m is 0 and Y is NH₂ or NHC₁₋₆alkyl.

Another preferred class of y-secretase inhibitors for use in this embodiment of the invention is that defined by formula XII: 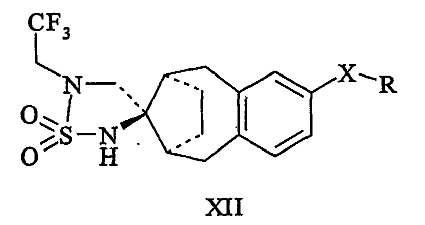 wherein X and R are as defined in WO 03/093252;
or a pharmaceutically acceptable salt thereof.

X is very aptly 5-substituted-thiazol-2-yl, 5-substitu.ted-4-methylthiazol-2-yl, 5-substituted-1-methylpyrazol-3-yl, 1-substituted-imidazol-4-yl or 1-substituted-1,2,4-triazol-3-yl. Preferably, R represents optionally-substituted phenyl or heteroaryl such as phenyl, monohalophenyl, dihalophenyl, trihalophenyl, cyanophenyl, methylphenyl, methoxyphenyl, trifluoromethylphenyl, trifluoromethoxyphenyl, pyridyl, monohalopyridyl and trifluoromethylpyridyl, wherein "halo" refers to fluoro or chloro. Particularly preferred identities of R-X- include 5-(4-fluorophenyl)-1-methylpyrazol-3-yl, 5-(4-chlorophenyl)-1-methylpyrazol-3-yl and 1-(4-fluorophenyl)imidazol-4-yl. Such compounds may be prepared by methods disclosed in WO 03/093232.

Further preferred classes of γ-secretase inhibitors include those disclosed in WO 03/093264, WO 03/093251, WO 03/093253, WO 2004/039370, WO 2004/39800 and WO 2004/031139.

Alternatively, the amyloid modifier may be a compound which inhibits the aggregation of Aβ. Suitable examples include chelating agents such as clioquinol (Gouras and Beal, Neuron, 30 (2001), 641-2) and the compounds disclosed in WO 99/16741, in particular that known as DP-109 (Kalendarev et al, J. Pharm. Biomed. Anal., 24 (2001), 967-75). Other inhibitors of Aβ aggregation suitable for use in the invention include the compounds disclosed in WO 96/28471, WO 98/08868 and WO 00/052048, including the compound known as Apan™ (Praecis); WO 00/064420, WO 03/017994, WO 99/59571 and the compound known as Alzhemed™ (Neurochem); WO 00/149281 and the compositions known as PTI-777 and PTI-00703 (ProteoTech); WO 96/39834, (WO 01/83425, WO 01/55093, WO 00/76988, WO 00/76987, WO 00/76969, WO 00/76489, WO 97/26919, WO 97/16194, and WO 97/16191.

Alternatively, the amyloid modifier may be an antibody which binds selectively to Aβ. Said antibody may be polyclonal or monoclonal, but is preferably monoclonal, and is preferably human or humanized. Preferably, the antibody is capable of sequestering soluble Aβ from biological fluids, as described in WO 03/016466, WO 03/016467, WO 03/015691 and WO 01/62801. Suitable antibodies include humanized antibody 266 (described in WO 01/62801) and the modified version thereof described in WO 03/016466.

As used herein, the expression "in combination with" requires that therapeutically effective amounts of both the compound of Formula I and the additional compound are administered to the subject, but places no restriction on the manner in which this is achieved. Thus, the two species may be combined in a single dosage form for simultaneous administration to the subject, or may be provided in separate dosage forms for simultaneous or sequential administration to the subject. Sequential administration may be close in time or remote in time, e.g. one species administered in the morning and the other in the evening. The separate species may be administered at the same frequency or at different frequencies, e.g. one species once a day and the other two or more times a day. The separate species may be administered by the same route or by different routes, e.g. one species orally and the other parenterally, although oral administration of both species is preferred, where possible. When the additional compound is an antibody, it will typically be administered parenterally and separately from the compound of Formula I.

In a further aspect, the invention provides a pharmaceutical composition comprising, in a pharmaceutically acceptable carrier, a compound of formula I or a pharmaceutically acceptable salt thereof and a compound of formula XI(a) or a pharmaceutically acceptable salt thereof. Preferably, the pharmaceutical composition is in a unit dose form suitable for oral administration, such as a tablet or a capsule.

### EXAMPLES

The ability of the compounds of Formula I to selectively inhibit production of Aβ(1-42) was determined using the following assay:

### Cell-based γ-Secretase Assay

Human SH-SY5Y neuroblastoma cells overexpressing the direct γ-secretase substrate SPA4CT were induced with sodium butyrate (10 mM) for 4 hours prior to plating. Cells were plated at 35,000 cells/well/100 µl in 96-well plates in phenol red-free MEM/10% FBS, 50 mM HEPES, 1% Glutamine and incubated for 2 hrs at 37 °C, 5% CO₂.

Compounds for testing were diluted into Me₂SO to give a ten point dose-response curve. Typically 10 µl of these diluted compounds in Me₂SO were further diluted into 182 µl dilution buffer (phenol red-free MEM/10% FBS, 50 mM HEPES, 1% Glutamine) and 10 µl of each dilution was added to the cells in 96-well plates (yielding a final Me₂SO concentration of 0.5%). Appropriate vehicle and inhibitor controls were used to determine the window of the assay.

After incubation overnight at 37 °C, 5% CO_{2,} 10 µl and 50 µl media were transferred into a fresh Costar round-bottom 96-well plate for detection of Aβ(40) and Aβ(42) peptides, respectively. 40 µl Origen buffer (PBS, 2% BSA, 0.2% Tween-20) was added to the Aβ(40) wells followed by the addition of 25 µl the respective antibody premixes to the wells:
Aβ(40) premix: 1 µg/ml ruthenylated G2-10 antibody, 4 µg/ml
biotinylated 4G8 antibody diluted in Origen buffer
Aβ(42) premix: 0.5 µg/ml ruthenylated G2-11 antibody, 4 µg/ml biotinylated 4G8 antibody diluted in Origen buffer

### (Biotinylated 4G8 antibody supplied by Signet Pathology Ltd; G2-10 and G2-11 antibodies supplied by Chemicon)

After overnight incubation of the assay plates on a shaker at 4°C, the Origen M8 Analyser (Igen Inc.) was calibrated according to the manufacturer's instructions. 25 µl of streptavidin magnetic bead (Dynal) premix (400 µg/ml streptavidin beads/ml in Origen buffer) was added to the assay plates and incubated on a shaker for 15 minutes. 150 µl Origen buffer was added to each well and the plates were read on the Origen M8 Analyser according to the manufacturer's instructions.

Cell viability was measured in the corresponding cells after removal of the media for the Aβ assays by a colorimetric cell proliferation assay (CellTiter 96™ AQ assay, Promega) utilizing the bioreduction of MTS (Owen's reagent) to formazan according to the manufacturer's instructions. Briefly, 5 µl of 10x MTS/PES was added to the remaining 50 µl of media before returning to the incubator. The optical density was read at 495 nm after ∼4 hours.

LD₅₀ and IC₅₀ values for inhibition of Aβ(40) and Aβ(42) were calculated by nonlinear regression fit analysis using the appropriate software (eg. Excel fit). The total signal and the background were defined by the corresponding Me₂SO and inhibitor controls.

The compounds listed in Tables 1 above all gave IC₅₀ values for Aβ(1-42) inhibition that were at least 2-fold lower than the corresponding IC₅₀ values for Aβ(1-40) inhibition, typically at least 5-fold lower, and in the preferred cases at least 50-fold lower.

All compounds were prepared by the methods disclosed in US 5,081,138; and the following provides a representative example:-

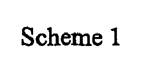

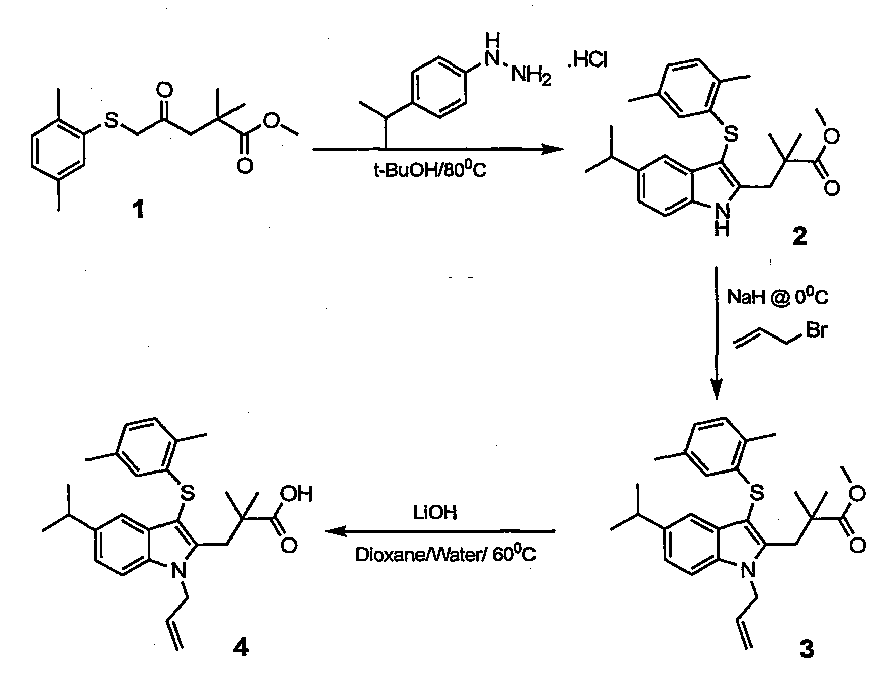

### Methyl 3-{3-[(2,5-dimethylphenyl)thiol-5-isopropyl-1H-indol-2-yl}-2,2-dimethylpropanoate (2)

A mixture of methyl 5-[(2,5-dimethylphenyl)thio]-2,2-dimethyl-4-oxopentanoate (1) (8.0g, 27.2mmol) [prepared from 5-bromo-2,2-dimethyl-4-oxopentanoate and 2,5-dimethylthiophenol using the method of US 5081138] and (4-isopropylphenyl)hydrazine hydrochloride (5.0g, 26.8mmol) in tert-butanol (50ml) was refluxed for 3hrs. Reaction mixture was cooled to ambient temperature and concentrated in vacuo. The residue was diluted with ethyl acetate (100ml) and sequentially washed with water (1x 100ml), hydrochloric acid (2N, 100ml) and brine (100ml). The organic extract was dried over MgSO₄, concentrated in vacuo, and purified by flash chromatography eluting with 2% ethyl acetate:hexane to afford methyl 3-{3-[(2,5-dimethylphenyl)thio]-5-isopropyl-1H-indol-2-yl}-2,2-dimethylpropanoate (2) as a off white solid (4.1g, 37%) ¹H NMR δ (ppm)(400MHz, CDCl₃):9.11(1H,s),7.32(2H,m),7.10(1H,dd,J=1.5,8.5Hz),7.01(1H,d,J= 7.5 Hz), 6.76 (1 H, d, J = 7.5 Hz), 6.45 (1 H, s), 3.73 (3 H, s), 3.14 (2 H, s), 3.00-2.90 (1 H, m), 2.46 (3 H, s), 2.03 (3 H, s), 1.20-1.27 (12 H, m).

### Methyl 3-{1-allyl-3-[(2,5-dimethylphenyl)thio]-5-isopropyl-1H-indol-2-yl}-2,2-dimethylpropanoate (3)

Sodium hydride (5mg, 0.22mmol) was added in to an ice cold solution of methyl 3- {3-[(2,5-dimethylphenyl)thio]-5-isopropyl-1H-indol-2-yl}-2,2-dimethylpropanoate (2) (60mg, 0.15mmol.) in DMF (3ml) and the resultant mixture stirred for 30min at 0°C. Then a solution of allyl bromide (26.6mg, 0.22mmol) in DMF (1ml) was added slowly and stirring was continued at 0°C for 30min and then at ambient temperature for 18hrs. The reaction mixture was diluted with water (50ml) and extracted with DCM (3x50ml). The organic extracts were washed with brine (1x100ml), dried over MgSO₄ and concentrated in vacuo. The residue was purified by flash chromatography eluting with 2% ethylacetate:hexane to afford methyl 3-{1-allyl-3-[(2,5-dimethylphenyl)thio]-5-isopropyl-1H-indol-2-yl}-2,2-dimethylpropanoate (3) (53mg, 80%).¹H NMR δ (ppm)( 360MHz, CDCl₃): 7.35 (1 H, s), 7.24 (1 H, m), 7.11 (1H, m), 7.00 (1 H, d, J = 7.5 Hz), 6.75 (1 H, d, J = 7.5 Hz), 6.36 (1 H, s), 5.94-5.84 (1 H, m), 5.12 (1 H, d, J = 10.5 Hz), 4.81-4.73 (3 H, m), 3.67 (3 H, s), 3.18 (2 H, s), 3.00-2.90 (1 H, m), 2.44 (3 H, s), 2.01 (3 H, s), 1.28-1.20 (12 H, m),

### 3-{1-allyl-3-[(2,5-dimethylphenyl)thio]-5-isopropyl-1H-indol-2-yl}-2,2-dimethylpropanoic acid (4)

A solution of lithium hydroxide (28mg, 1.2mmol) in water (1ml) was added into a solution methyl 3-{1-allyl-3-[(2,5-dimethylphenyl)thio]-5-isopropyl-1 H -indol-2-yl}-2,2-dimethylpropanoate (3) (53mg, 0.12mmol) in dioxane (2ml) and stirred at 60°C for 18hrs. The reaction mixture was diluted with hydrochloric acid (2N, 50ml) and extracted with ethyl acetate (2x50ml). The organic extract was washed with brine (1x50ml), dried over MgSO₄ and concentrated in vacuo, to afford 3-{1-allyl-3-{(2,5-dimethylphenyl)thio]-5-isopropyl-1 H -indol-2-yl}-2,2-dimethylpropanoic acid (41mg, 78%) 1H NMR δ (ppm)( 360MHz, CDCl3): 7.36 (1 H, s), 7.25 (1H, m), 7.12 (1 H, dd, J = 1.5, 8.5 Hz), 7.00 (1 H, d, J = 7.5 Hz), 6.75 (1 H, d, J = 7.5 Hz), 6.37 (1 H, s), 5.94-5.86 (1 H, m), 5.12 (1 H, d, J =10.5 Hz), 4.85 (2 H,m), 4.77 (1 H, d, J =17.0 Hz), 3.22 (2 H, s), 3.01-2.91 (1 H, m), 2.44 (3 H, s), 2.00 (3 H, s), 1.23-1.27 (12 H, m).

## Claims

1. The use, for the manufacture of a medicament for selective attenuation of the production of the (1-42) isoform of Aβ, of a compound of Formula I 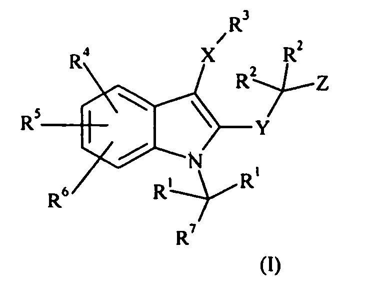 wherein each R¹ is independently H, C₁₋₆alkyl or C₃₋₆cycloalkyl;
each R² is independently H or C₁₋₆alkyl, or the two R² groups together complete a C₃₋₆cycloalkyl group;
R³ represents a hydrocarbon group of up to 10 carbon atoms optionally bearing up to 2 substituents selected from halogen, CN, CF₃, OCF₃, C₁₋₄alkoxy, C₁₋₄alkylthio, CO₂H, C₁₋₄alkoxycarbonyl, C₁₋₄alkylcarbonyl and C₁₋₄alkylcarbonyloxy; or R³ represents a heteroaryl group of 5 or 6 ring atoms which is optionally benzo-fused, optionally bearing up to 2 substituents selected from C₁₋₄alkyl, halogen, CN, CF₃, OCF₃, C₁₋₄alkoxy, C₁₋₄alkylthio, CO₂H, C₁₋₄alkoxycarbonyl,C₁₋₄alkylcarbonyl and C₁₋₄ alkylcarbonyloxy;
R^{4,} R⁵ and R⁶ independently represent H, halogen, CN, CF₃, OCF₃, C₁₋₄alkoxy, C₁₋₄alkylthio, CO₂H, C₁₋₄alkoxycarbonyl, C₁₋₄alkylcarbonyl, C₁₋₄alkylcarbonyloxy or hydrocarbon of up to 7 carbon atoms optionally bearing a substituent selected from halogen, CN, CF₃, OCF₃, C₁₋₄alkoxy, C₁₋₄alkylthio, CO₂H, C₁₋₄alkoxycarbonyl, C₁₋₄alkylcarbonyl and C₁₋₄alkylcarbonyloxy;
R⁷ represents a hydrocarbon group of up to 10 carbon atoms optionally bearing up to 2 substituents selected from halogen, CN, CF₃, OCF₃, C₁₋₄alkoxy, C₁₋₄alkylthio, CO₂H, C₁₋₄alkoxycarbonyl, C₁₋₄alkylcarbonyl and C₁₋₄alkylcarbonyloxy;
X represents O or S;
Y represents a bond, CH₂, CH₂CH₂, CH₂CH₂CH₂ or CH₂CH₂O; and
Z represents CO₂H or tetrazol-5-yl;
or a pharmaceutically acceptable salt thereof.

2. Use according to claim 1 wherein said medicament is for administration to a patient suffering from a disease selected from Alzheimer's disease, cerebral amyloid angiopathy, HCHWA-D, multi-infarct dementia, dementia puglistica and Down syndrome.

3. Use according to claim 1 in which the medicament is for administration to a patient suffering from mild cognitive impairment or age related cognitive decline.

4. Use, for the manufacture of a medicament for retarding, arresting or preventing the accumulation of Aβ in the brain, of a compound of Formula I as defined in claim 1 or a pharmaceutically acceptable salt thereof.

5. Use according to any previous claim wherein X is S.

6. Use according to any previous claim wherein Y is CH₂.

7. Use according to any previous claim wherein Z is CO₂H

8. Use according to any previous claim wherein R³ in formula I is selected from alkyl, cycloalkyl, alkyl-substituted phenyl and chloro-substituted phenyl.

9. Use according to any previous claim wherein R⁷ in formula I is selected from alkyl, cycloalkyl or alkenyl of up to 6 carbon atoms, or optionally substituted phenyl.

10. Use according to claim 1 of a compound according to formula II: 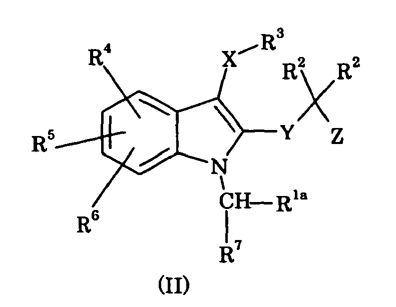 wherein R^{1a} represents C₁₋₆alkyl or C₃₋₆cycloalkyl, and R²-R⁷, X, Y and Z are as defined in claim 1;
or a pharmaceutically acceptable salt thereof.

11. Use according to claim 1 of a compound according to formula III: 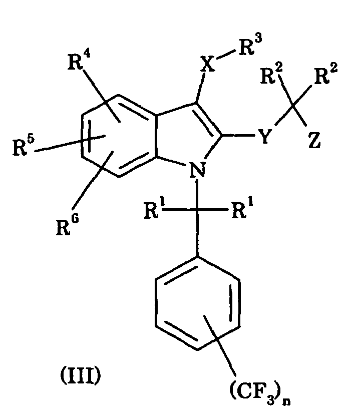 wherein n is 1 or 2 and R¹-R⁶, X, Y and Z are as defined in claim 1;
or a pharmaceutically acceptable salt thereof.

12. A compound of formula II 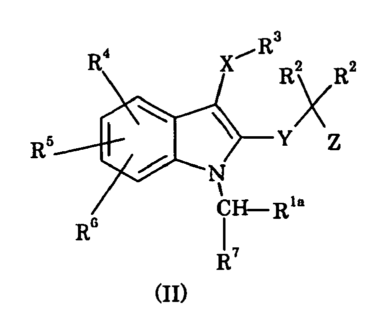 wherein R^{1a} represents C₁₋₆alkyl or C₃₋₆cycloalkyl, and R²-R⁷, X, Y and Z are as defined in claim 1;
or a pharmaceutically acceptable salt thereof.

13. A compound of formula III: 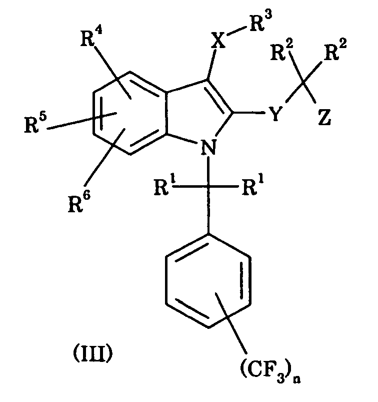 wherein n is 1 or 2 and R¹-R⁶, X, Y and Z are as defined in claim 1;
or a pharmaceutically acceptable salt thereof.

14. A pharmaceutical composition comprising, in a pharmaceutically acceptable carrier, a compound of formula II or formula III, or a pharmaceutically acceptable salt thereof.

## Patentansprüche

1. Die Verwendung zur Herstellung eines Medikaments zur selektiven Verminderung der Produktion der (1-42)-Isoform von Aβ einer Verbindung der Formel I 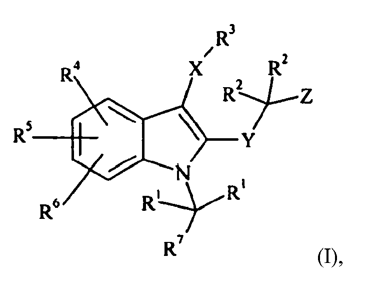 wobei jedes R¹ unabhängig H, C₁₋₆-Alkyl oder C₃₋₆-Cycloalkyl ist,
jedes R² unabhängig H oder C₁₋₆-Alkyl ist, oder die zwei R²-Gruppen zusammen eine C₃₋₆-Cycloalkylgruppe vervollständigen,
R³ eine Kohlenwasserstoffgruppe mit bis zu 10 Kohlenstoffatomen, gegebenenfalls bis zu 2 Substituenten tragend, ausgewählt aus Halogen, CN, CF₃, OCF₃, C₁₋₄-Alkoxy, C₁₋₄-Alkylthio, CO₂H, C₁₋₄-Alkoxycarbonyl, C₁₋₄-Alkylcarbonyl und C₁₋₄Alkylcarbonyloxy, bedeutet, oder R³ eine Heteroarylgruppe mit 5 oder 6 Ringatomen bedeutet, die gegebenenfalls benzokondensiert ist, gegebenenfalls bis zu 2 Substituenten tragend, ausgewählt aus C₁₋₄-Alkyl, Halogen, CN, CF₃, OCF₃, C₁₋₄-Alkoxy, C₁₋₄-Alkylthio, CO₂H, C₁₋₄-Alkoxycarbonyl, C₁₋₄-Alkylcarbonyl und C₁₋₄-Alkylcarbonyloxy,
R⁴, R⁵ und R⁶ unabhängig H, Halogen, CN, CF₃, OCF₃, C₁₋₄-Alkoxy, C₁₋₄-Alkylthio, CO₂H, C₁₋₄-Alkoxycarbonyl, C₁₋₄-Alkylcarbonyl, C₁₋₄-Alkylcarbonyloxy oder Kohlenwasserstoff mit bis zu 7 Kohlenstoffatomen, gegebenenfalls einen Substituenten tragend, ausgewählt aus Halogen, CN, CF₃, OCF₃, C₁₋₄-Alkoxy, C₁₋₄-Alkylthio, CO₂H, C₁₋₄-Alkoxycarbonyl, C₁-₄-Alkylcarbonyl und C₁₋₄-Alkylcarbonyloxy, bedeuten,
R⁷ eine Kohlenwasserstoffgruppe mit bis zu 10 Kohlenstoffatomen, gegebenenfalls bis zu 2 Substituenten tragend, ausgewählt aus Halogen, CN, CF₃, OCF₃, C₁₋₄-Alkoxy, C₁₋₄-Alkylthio, CO₂H, C₁₋₄-Alkoxycarbonyl, C₁₋₄-Alkylcarbonyl und C₁₋₄-Alkylcarbonyloxy, bedeutet,
X O oder S bedeutet,
Y eine Bindung, CH₂, CH₂CH₂, CH₂CH₂CH₂ oder CH₂CH₂O bedeutet, und
Z CO₂H oder Tetrazol-5-yl bedeutet,
oder ein pharmazeutisch annehmbares Salz davon.

2. Verwendung gemäß Anspruch 1, wobei das Medikament zur Verabreichung an einen Patienten dient, der an einer Erkrankung leidet, ausgewählt aus Alzheimer-Krankheit, zerebraler Amyloidangiopathie, HCHWA-D, Multi-Infarkt-Demenz, Dementia puglistica und Down-Syndrom.

3. Verwendung gemäß Anspruch 1, wobei das Medikament zur Verabreichung an einen Patienten dient, der an leichter kognitiver Beeinträchtigung oder an altersbedingtem Abbau der kognitiven Fähigkeiten leidet.

4. Verwendung einer wie in Anspruch 1 definierten Verbindung der Formel I oder eines pharmazeutisch annehmbaren Salzes davon zur Herstellung eines Medikaments zur Verzögerung, Hemmung oder Verhinderung der Anreicherung von Aβ im Gehirn.

5. Verwendung gemäß einem vorherigen Anspruch, wobei X S ist.

6. Verwendung gemäß einem vorherigen Anspruch, wobei Y CH₂ ist.

7. Verwendung gemäß einem vorherigen Anspruch, wobei Z CO₂H ist.

8. Verwendung gemäß einem vorherigen Anspruch, wobei R³ in Formel I ausgewählt ist aus Alkyl, Cycloalkyl, alkylsubstituiertem Phenyl und chlorsubstituiertem Phenyl.

9. Verwendung gemäß einem vorherigen Anspruch, wobei R⁷ in Formel I ausgewählt ist aus Alkyl, Cycloalkyl oder Alkenyl mit bis zu 6 Kohlenstoffatomen oder gegebenenfalls substituiertem Phenyl.

10. Verwendung gemäß Anspruch 1 einer Verbindung gemäß Formel II: 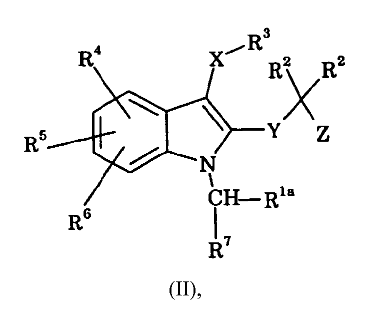 wobei R^{1a} C₁₋₆-Alky1 oder C₃₋₆-Cycloalkyl bedeutet und R² -R⁷, X, Y und Z wie in Anspruch 1 definiert sind, oder eines pharmazeutisch annehmbaren Salzes davon.

11. Verwendung gemäß Anspruch 1 einer Verbindung gemäß Formel III: 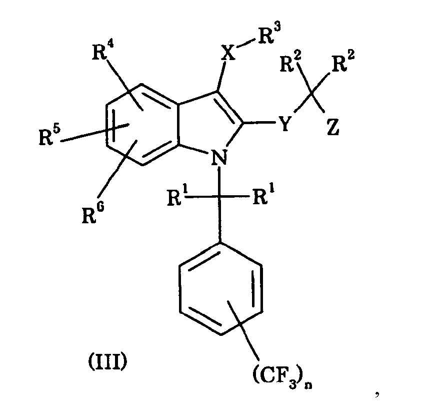 wobei n 1 oder 2 ist und R¹-R⁶, X, Y und Z wie in Anspruch 1 definiert sind,
oder eines pharmazeutisch annehmbaren Salzes davon.

12. Eine Verbindung der Formel II 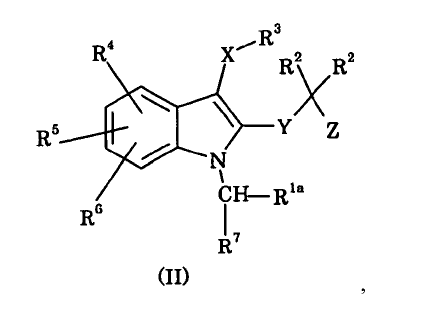 wobei R^{1a} C₁₋₆-Alkyl oder C₃₋₆-Cycloalkyl bedeutet und R² -R⁷, X, Y und Z wie in Anspruch 1 definiert sind, oder ein pharmazeutisch annehmbares Salz davon.

13. Eine Verbindung der Formel III: 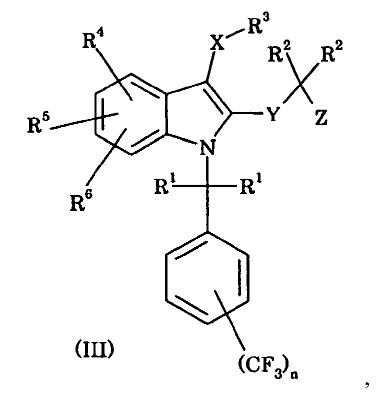 wobei n 1 oder 2 ist und R¹-R⁶, X, Y und Z wie in Anspruch 1 definiert sind,
oder ein pharmazeutisch annehmbares Salz davon.

14. Eine pharmazeutische Zusammensetzung, die in einem pharmazeutisch annehmbaren Träger eine Verbindung der Formel II oder Formel III oder ein pharmazeutisch annehmbares Salz davon enthält.

## Revendications

1. Utilisation, pour la fabrication d'un médicament pour une atténuation sélective de la production de l'isoforme (1-42) de Aβ, d'un composé de la Formule I: 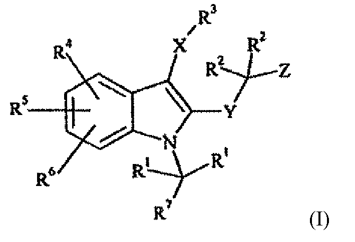 dans laquelle chaque R¹ est indépendamment H, un alkyle C₁₋₆ ou un cycloalkyle C₃-₆;
chaque R² est indépendamment H ou un alkyle C₁₋₆, ou les deux groupes R² ensemble complètent un groupe cycloalkyle C₃₋₆;
R³ représente un groupe hydrocarbure de jusqu'à 10 atomes de carbone portant optionnellement jusqu'à 2 substituants choisis parmi un halogène, CN, CF₃, OCF₃, un alcoxy C₁₋₄, un alkylthio C₁₋₄, CO₂H, un alcoxycarbonyle C₁₋₄, un alkylcarbonyle C₁₋₄ et un alkylcarbonyloxy C₁₋₄; ou R³ représente un groupe hétéroaryle de 5 ou 6 atomes de cycle qui est optionnellement condensé par un benzo, portant optionnellement jusqu'à 2 substituants choisis parmi un alkyle C₁₋₄, un halogène, CN, CF₃, OCF₃, un alcoxy C₁₋₄, un alkylthio C₁₋₄, CO₂H, un alcoxycarbonyle C₁₋₄, un alkylcarbonyle C₁₋₄ et un alkylcarbonyloxy C₁₋₄;
R⁴, R⁵ et R⁶ représentent indépendamment H, un halogène, CN, CF₃, OCF₃, un alcoxy C₁₋₄, un alkylthio C₁₋₄, CO₂H, un alcoxycarbonyle C₁₋₄, un alkylcarbonyle C₁₋₄, un alkylcarbonyloxy C₁₋₄ ou un hydrocarbure de jusqu'à 7 atomes de carbone portant optionnellement un substituant choisi parmi un halogène, CN, CF₃, OCF₃, un alcoxy C₁₋₄, un alkylthio C₁₋₄, CO₂H, un alcoxycarbonyle C₁₋₄, un alkylcarbonyle C₁₋₄ et un alkylcarbonyloxy C₁₋₄;
R⁷ représente un groupe hydrocarbure de jusqu'à 10 atomes de carbone portant optionnellement jusqu'à 2 substituants choisis parmi un halogène, CN, CF₃, OCF₃, un alcoxy C₁₋₄, un alkylthio C₁₋₄, CO₂H, un alcoxycarbonyle C₁₋₄, un alkylcarbonyle C₁₋₄ et un alkylcarbonyloxy C₁₋₄;
X représente O ou S;
Y représente une liaison, CH₂, CH₂CH₂, CH₂CH₂CH₂ ou CH₂CH₂O; et
Z représente CO₂H ou un tétrazol-5-yle;
ou d'un sel pharmaceutiquement acceptable de celui-ci.

2. Utilisation selon la revendication 1, dans laquelle ledit médicament est pour une administration à un patient souffrant d'une maladie choisie parmi une maladie d'Alzheimer, une angiopathie amyloïde cérébrale, HCHWA-D, une démence par infarctus multiples, une dementia puglistica et un syndrome de Down.

3. Utilisation selon la revendication 1, dans laquelle le médicament est pour une administration à un patient souffrant d'une déficience intellectuelle modérée ou d'un déclin intellectuel lié à l'âge.

4. Utilisation, pour la fabrication d'un médicament pour retarder, arrêter ou prévenir l'accumulation de Aβ dans le cerveau, d'un composé de la Formule I telle que définie dans la revendication 1 ou d'un sel pharmaceutiquement acceptable de celui-ci.

5. Utilisation selon l'une quelconque des revendications précédentes, dans laquelle X est S.

6. Utilisation selon l'une quelconque des revendications précédentes, dans laquelle Y est CH₂.

7. Utilisation selon l'une quelconque des revendications précédentes, dans laquelle Z est CO₂H.

8. Utilisation selon l'une quelconque des revendications précédentes, dans laquelle R³ dans la Formule I est choisi parmi un alkyle, un cycloalkyle, un phényle alkyle-substitué et un phényle chloro-substitué.

9. Utilisation selon l'une quelconque des revendications précédentes, dans laquelle R⁷ dans la Formule I est choisi parmi un alkyle, un cycloalkyle ou un alcényle de jusqu'à 6 atomes de carbone ou un phényle optionnellement substitué.

10. Utilisation selon la revendication 1 d'un composé selon la Formule II: 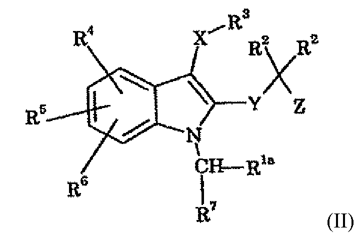 dans laquelle R^{1a} représente un alkyle C₁₋₆ ou un cycloalkyle C₃₋₆, et R²-R⁷, X, Y et Z sont tels que définis dans la revendication 1;
ou d'un sel pharmaceutiquement acceptable de celui-ci.

11. Utilisation selon la revendication 1 d'un composé selon la Formule III: 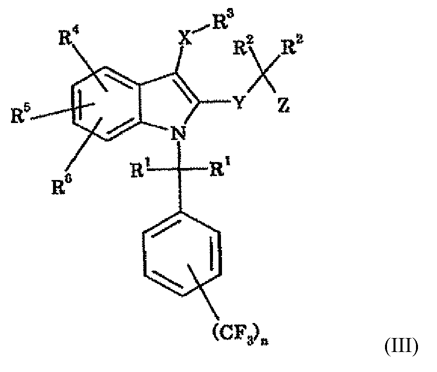 dans laquelle n est 1 ou 2 et R¹-R⁶, X, Y et Z sont tels que définis dans la revendication 1;
ou d'un sel pharmaceutiquement acceptable de celui-ci.

12. Composé de la Formule II: 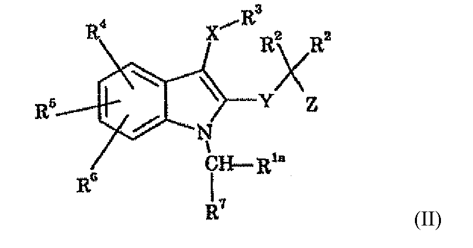 dans laquelle R^{1a} représente un alkyle C₁₋₆ ou un cycloalkyle C₃₋₆, et R²-R⁷, X, Y et Z sont tels que définis dans la revendication 1;
ou sel pharmaceutiquement acceptable de celui-ci.

13. Composé de la Formule III: 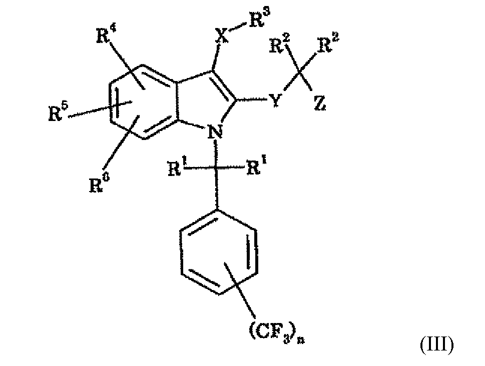 dans laquelle n est 1 ou 2 et R¹-R⁶, X, Y et Z sont tels que définis dans la revendication 1;
ou sel pharmaceutiquement acceptable de celui-ci.

14. Composition pharmaceutique comprenant, dans un support pharmaceutiquement acceptable, un composé de la Formule II ou de la Formule III, ou un sel ρharmaceutiquement acceptable de celui-ci.
